# EUROPEAN PATENT SPECIFICATION

(11) **EP 1 373 225 B1**
(45) Date of publication and mention of the grant of the patent: **24.05.2006**
(21) Application number: 02763863.4
(22) Date of filing: 22.03.2002
(51) Int. Cl.: C07D 239/84, C07D 487/04, C07D 475/04

(54) **PROCESS FOR COUPLING AMINO ACIDS TO AN ANTIFOLATE SCAFFOLD**
VERFAHREN ZUR KUPPLUNG VON AMINOSÄUREN AN DAS GERÜST EINES FOLSÄUREANTAGONISTEN
PROCEDE DE COUPLAGE D'ACIDES AMINES A UN SQUELETTE ANTI-ACIDE FOLIQUE

(30) Priority: 04.04.2001 US 281451 P
(43) Date of publication of application: 02.01.2004
(73) Proprietor: BIONUMERIK PHARMACEUTICALS, INC., San Antonio, Texas 78229 (US)
(72) Inventor: KOCHAT, Harry, San Antonio, TX 78248 (US); WU, Ye, Helotes, TX 78023 (US)
(74) Representative: Lucas, Brian Ronald
(86) International application number: PCT/US2002/008932
(87) International publication number: WO 2002/081455

(56) References cited:
- EP-A- 0 589 720
- WO-A-91/10666
- US-A- 5 550 128
- US-A- 5 698 556
- ABRAHAM, ANN ET AL: "Folate analogs. 34. Synthesis and antitumor activity of non-polyglutamylatable inhibitors of dihydrofolate reductase" JOURNAL OF MEDICINAL CHEMISTRY , 34(1), 222-7 CODEN: JMCMAR; ISSN: 0022-2623, 1991, XP002295839
- A. ROSOWSKY ET AL.: "Analogues of Methothrexate and Aminopterin with gamma-methylene and gamma-cyano substitution of the glutamate side chain: Synthesis and in vitro biological activity" J. MED. CHEM., vol. 34, no. 1, 1991, pages 203-208, XP002295840

## Description

This invention relates to a novel process for coupling amino acids to a substrate, and will have special application in the synthetic processes for synthesizing antifolate compounds.

Antifolates are a well-known class of compounds that heretofore have been useful in treating various cancers and infectious diseases in humans and animals. They derive their classification based on their effectiveness in disrupting or otherwise antagonizing the folic acid metabolic pathway.

Structurally, antifolates have been classed as 'classical' and 'non-classical.' So-called classical antifolates resemble the structure of folic acid in that they include a fused ring moiety, which is bonded to an aromatic moiety, which in turn is bonded to an amino acid. The structures of the classical antifolate drugs methotrexate, aminopterin, MDAM and M-Trex, are shown below.

Synthesis of most classical antifolates is often an arduous process. The molecules are not only complex, but many of the intermediates are poorly soluble in commonly used solvents, further complicating the synthesis and often resulting in poor yields and/or low purity of the intended compound.

The currently known coupling processes for unsaturated amino acids (e.g., γ-methylene glutamic acid and esters thereof) are hampered by the poor solubility profile of the unsaturated amino acid. United States Patent 4,996,207, and the publications attached to the Information Disclosure Sheet disclose the prior preferred process for coupling the unsaturated amino acid residue to a pteroic acid, or equivalent scaffold.

As disclosed therein, the prior coupling processes involved the use of dimethyl formamide (DMF), a highly polar, protonated solvent. DMF is a highly toxic, chemically reactive, flammable solvent that poses certain health hazards in practical laboratory use, and requires the technician to wear suitable protective clothing as well as taking precautions against inhalation of the vapors.

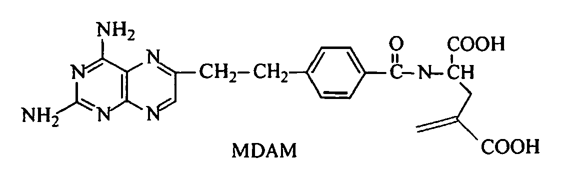

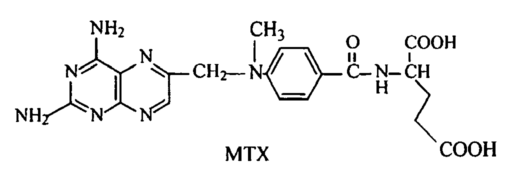

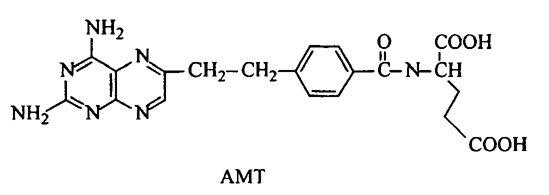

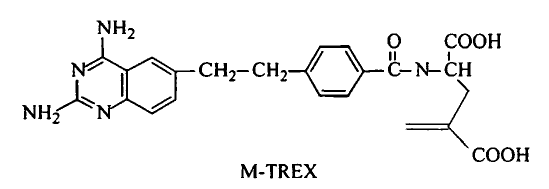

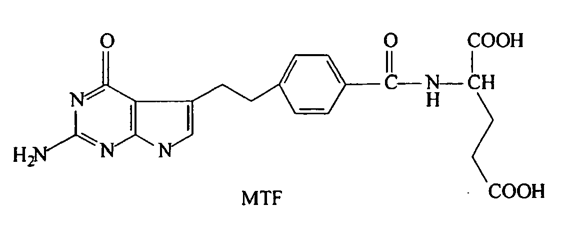

### SUMMARY OF THE INVENTION

The coupling process of this invention utilizes, N-Methylpyrrolidinone to dissolve the reagents used in coupling an amino acid to a scaffold to form a classical antifolate. The amino acid to be coupled is preferably unsaturated, and relatively poorly water soluble.

The process is particularly useful to form antifolates having the following formula (I): X-R₁-R₂-CO-A, where X is a fused ring heterocyclic system; R₁ is lower alkylene optionally substituted by one or more lower alkyl, halogen, aryl or heterocycle moieties for a corresponding hydrogen atom; or -CR₃R₄-NR₅-; R₂ is arylene or a heterocyclylene; R₃, R₄ and R₅ are individually hydrogen, lower alkyl, aryl, amido or a protecting group; and A is the amino acid.

Together, the four parts of the formula (I) compound combine to form what is defined herein as a "classical" antifolate compound, useful in the treatment of cancer and various autoimmune diseases of humans and animals.

The coupling process of this invention involves dissolving the amino acid and the scaffold in the nonpolar, aprotic solvent, adding a coupling reagent and agitating the resulting mixture for a predetermined time period. The formula I compound is then precipitated by conventional means and analyzed for purity.

Accordingly, it is an object of this invention to provide for an efficient process for synthesizing classical antifolates.

Another object of this invention is to provide for a coupling process that boosts yield and purity of the desired antifolate end product.

Other objects will become apparent upon a reading of the following specification.

### DESCRIPTION OF THE PREFERRED EMBODIMENT

The preferred embodiment herein described is not intended to be exhaustive or to limit the invention to the precise form disclosed. It is chosen and described to explain the principles of the invention and its application and practical use to enable others skilled in the art to practice its teachings.

The process of this invention, which involves the coupling of an amino acid to a scaffold to produce a classical antifolate I, is disclosed in the following Scheme.

X-R₁-R₂-COOH (X) + A → X-R₁-R₂-CO-A (I)

As shown, the process is a single step process that couples the amino acid A to the scaffold X. The scaffold X can be any commonly known system that forms a classical antifolate, including but not limited to the types of structures shown above. The amino acid A is preferably in ester form, most preferably a glutamic acid residue, or unsaturated analog, or salt thereof, as is preferred for most antifolates.

The process is effectuated by dissolving the scaffold in N-Methylpyrrolidinone. The amino acid is then added to the mixture, preferably along with a coupling reagent and a stereoselectivity reagent, and the mixture is agitated for a predetermined time period. The formula I compound is then precipitated out of solution and purified using known methods.

Preferred coupling reagents include N-(3-dimethylaminopropyl) N'-ethyl carbodiimide (EDC), and the like and are commonly known reagents, available through commercial sources.

The stereoselectivity reagent is useful to prevent racemization of the amino acid, with preferred reagents including the benzotriazoles, most preferably N-hydroxy benzotriazole. Since the L-enantiomer of an amino acid is generally known to be the active enantiomer for medicinal purposes, addition of this reagent ensures that the amino acid remains in its active form throughout the process.

The process is preferably carried out in a basic solution by adding a small amount of an organic base, such as triethylamine or the like. Preferred pH of the reaction mixture is 7.5-9.0.

The examples below illustrate the process as employed to couple an unsaturated derivatized amino acid to a scaffold, producing a desired antifolate agent. The examples, including reagents used and reaction conditions are not to be considered as limiting the invention to those specific reagents or conditions.

### EXAMPLE 1

1.1 grams of pteroic acid was weighed into a 25 mL round bottom flask equipped with a nitrogen purge and a magnetic stirrer. 26 mL of anhydrous NMP was added to the flask to form a suspension, which was stirred for 10 minutes at room temperature. 1.25 grams of gamma methylene glutamic acid hydrochloride, 110 mg of N-hydroxy benzotriazole, and 1.36 grams of EDC were then added to the mixture and stirring continued for 30 minutes. 1 mL of triethyl amine was then added to the mixture, which was then stirred for 16 hours.

An aliquot was withdrawn, precipitated with water and analyzed by HPLC to ensure complete disappearance of the starting material.

The reaction mixture was then poured over crushed ice and allowed to stand for 1-2 hours. The resulting precipitate was then filtered and washed with 75% ethanol and 25% ether mixture. The fine precipitate obtained was then dried under high vacuum and NMR recorded.

The precipitate was then taken up in saturated bicarbonate/ethanol (50/50) and stirred for 1 hour at room temperature. The precipitate was then filtered, washed with ethanol, dried and again analyzed by NMR and HPLC. Yield was 1.428 grams (94.1%) at 97.1% purity.

¹H NMR: δ 1.05-1.3 (6H, t, J=7.3); 2. 6 (1H,s); 2. 61 (1H,m); 2.82 (1H,m); 3.4 (2H,m); 4.01 (4H,m); 4.52 (1H,m 5.61 (1H,m); 6.05 (1H,m); 6.5 (2H,m); 7.65 (2H,m); 7.56 (2H,m); 7.7 (2H,m); 8.44 (1H,s); 8.6 (1H,m).

Similar procedures may be used in coupling other amino acids to an antifolate scaffold by varying the starting reagents. The above specification is not limiting of the invention to the precise details, but may be modified within the scope of the following claims.

## Claims

1. A process for synthesizing an antifolate compound having the formula (I) by coupling an unsaturated amino acid, or an ester or a salt thereof, to a scaffold, the process comprising dissolving the amino acid, or ester or salt, and the scaffold in N-methylpyrrolidinone as a solvent; and agitating the resulting mixture for a predetermined time to form the formula (I) compound:
X-R₁-R₂-CO-A (I)
wherein X-R₁-R₂-CO is a residue of the scaffold X-R₁-R₂-COOH, and X is an aromatic fused ring heterocyclic system; R₁ is lower alkylene or a lower alkylene in which one or more hydrogen atoms are substituted respectively with one or more lower alkyl, halogen, aryl or heterocycle moieties, or R₁ is ―CH₂NR₃―; R₂ is arylene, cycloalkylene or heterocyclylene; R₃ is hydrogen, lower alkyl, lower alkenyl, or lower alkynyl, or a substituted analogue thereof; and A is an unsaturated amino acid or unsaturated amino acid ester or salt residue.

2. The process of Claim 1 wherein the amino acid is gamma-methylene glutamic acid or an ester or a salt thereof.

3. The process of Claim 2 wherein the amino acid is L-gamma methylene glutamic acid or an ester or a salt thereof.

4. The process of Claim 1 wherein X is a two-ring fused system with one or more ring atoms being nitrogen.

5. The process of any of Claims 1 to 4 wherein a coupling reagent and a stereoselectivity reagent are present.

6. The process of Claim 5 wherein the coupling reagent is N-(3-dimethylaminopropyl) N'-ethyl carbodiimide.

7. The process of Claim 5 wherein the stereoselectivity reagent is a benzotriazole.

8. The process of Claim 7 wherein the stereoselectivity reagent is N-hydroxy benzotriazole.

9. The process of Claim 1 wherein an enantiomer of the unsaturated amino acid is coupled to the scaffold in the presence of a stereoselectivity reagent.

10. The process of Claim 9 wherein the enantiomer is the L-enantiomer.

## Patentansprüche

1. Verfahren zum Synthetisieren einer Folsäureantagonisten-Verbindung mit der Formel (I) durch Kupplung einer ungesättigten Aminosäure oder eines Esters oder eines Salzes davon an ein Gerüst, wobei das Verfahren das Lösen der Aminosäure oder des Esters oder des Salzes und des Gerüsts in N-Methylpyrrolidinon als Lösungsmittel und das Rühren des entstandenen Gemischs für eine vorbestimmte Zeit umfaßt, wodurch die Verbindung der Formel (I) gebildet wird:
X-R₁-R₂-CO-A (I)
worin X-R₁-R₂-CO ein Rest des Gerüsts X-R₁-R₂-COOH ist und X ein aromatisches kondensiertes heterocyclisches Ringsystem ist; R₁ Niederalkylen oder ein Niederalkylen ist, bei ein oder mehrere Wasserstoffatome durch ein oder mehrere Niederalkyl-, Halogen-, Aryl- oder heterocyclische Einheiten substituiert ist bzw. sind, oder R₁ -CH₂NR₃- ist; R₂ Arylen, Cycloalkylen oder Heterocyclylen ist; R₃ Wasserstoff, Niederalkyl, Niederalkenyl oder Niederalkinyl oder ein substituiertes Analog davon ist; und A eine ungesättigte Aminosäure oder ein ungesättigter Aminosäureester oder Salzrest ist.

2. Verfahren nach Anspruch 1, wobei die Aminosäure γ-Methylenglutaminsäure oder ein Ester oder ein Salz davon ist.

3. Verfahren nach Anspruch 2, wobei die Aminosäure L-γ-Methylenglutaminsäure oder ein Ester oder ein Salz davon ist.

4. Verfahren nach Anspruch 1, wobei X ein System aus zwei kondensierten Ringen ist, wobei ein oder mehrere Ringatome Stickstoff ist bzw. sind.

5. Verfahren nach einem der Ansprüche 1 bis 4, wobei ein Kupplungsreagenz und ein Stereoselektivitätsreagenz vorliegen.

6. Verfahren nach Anspruch 5, wobei das Kupplungsreagenz N-(3-Dimethylaminopropyl)-N'-ethylcarbodiimid ist.

7. Verfahren nach Anspruch 5, wobei das Stereoselektivitätsreagenz ein Benzotriazol ist.

8. Verfahren nach Anspruch 7, wobei das Stereoselektivitätsreagenz N-Hydroxybenzotriazol ist.

9. Verfahren nach Anspruch 1, wobei ein Enantiomer der ungesättigten Aminosäure in Gegenwart eines Stereoselektivitätsreagenz an das Gerüst gekuppelt wird.

10. Verfahren nach Anspruch 9, wobei das Enantiomer das L-Enantiomer ist.

## Revendications

1. Procédé de synthèse d'un composé antifolate de formule (I) par couplage d'un acide aminé insaturé, ou d'un ester ou d'un sel de celui-ci, à un squelette, ce procédé comprenant la dissolution de l'acide aminé, ou de l'ester ou du sel, et du squelette dans de la N-méthylpyrrolidinone en tant que solvant ; et l'agitation du mélange obtenu pendant une durée prédéterminée pour former le composé de formule (I) :
X-R₁-R₂-CO-A (I)
dans laquelle X-R₁-R₂-CO représente un résidu du squelette X-R₁-R₂-COOH, et X représente un système hétérocyclique à cycle condensé aromatique ; R₁ représente un groupe alkylène inférieur ou un groupe alkylène inférieur dans lequel un ou plusieurs atomes d'hydrogène sont respectivement substitués par un ou plusieurs alkyles inférieurs, halogènes, aryles ou fractions hétérocycliques, ou R₁ représente -CH₂NR₃- ; R₂ représente un groupe arylène, cycloalkylène ou hétérocyclylène ; R₃ représente un hydrogène, un alkyle inférieur, un alcényle inférieur ou un alcynyle inférieur, ou un analogue substitué de ceux-ci ; et A représente un résidu d'acide aminé insaturé ou de sel ou d'ester d'acide aminé insaturé.

2. Procédé selon la revendication 1, dans lequel l'acide aminé est l'acide gamma-méthylène-glutamique ou un ester ou un sel de celui-ci.

3. Procédé selon la revendication 2, dans lequel l'acide aminé est l'acide L-gamma-méthylène-glutamique ou un ester ou un sel de celui-ci.

4. Procédé selon la revendication 1, dans lequel X représente un système condensé à deux cycles, un ou plusieurs atomes cycliques étant des atomes d'azote.

5. Procédé selon l'une quelconque des revendications 1 à 4, dans lequel un réactif de couplage et un réactif de stéréosélectivité sont présents.

6. Procédé selon la revendication 5, dans lequel le réactif de couplage est le N-(3-diméthylaminopropyl)N'-éthyl-carbodiimide.

7. Procédé selon la revendication 5, dans lequel le réactif de stéréosélectivité est un benzotriazole.

8. Procédé selon la revendication 7, dans lequel le réactif de stéréosélectivité est le N-hydroxy-benzotriazole.

9. Procédé selon la revendication 1, dans lequel un énantiomère de l'acide aminé insaturé est couplé au squelette en présence d'un réactif de stéréosélectivité.

10. Procédé selon la revendication 9, dans lequel l'énantiomère est l'énantiomère L.
